# EUROPEAN PATENT APPLICATION

(11) **EP 1 938 811 A1**
(43) Date of publication of application: **02.07.2008**
(21) Application number: 06810451.2
(22) Date of filing: 22.09.2006
(51) Int. Cl.: A61K 31/137, A61K 31/27, A61K 31/407, A61K 31/445, A61K 31/473, A61K 31/553, A61K 31/662, A61K 45/00, A61P 3/04, A61P 25/18, A61P 25/24, A61P 43/00

(54) **MEDICINAL COMPOSITION FOR TREATING BULIMIA AND DEPRESSION ACCOMPANYING BULIMIA**

(30) Priority: 22.09.2005 JP 2005276155
(71) Applicant: Eisai R&D Management Co., Ltd., Tokyo 112-8088 (JP)
(72) Inventor: SAKAI, Kazuo, Tokyo 112-0002 (JP)
(74) Representative: HOFFMANN EITLE
(86) International application number: PCT/JP2006/318838
(87) International publication number: WO 2007/034910

(57) **Abstract**

Provided is a pharmaceutical composition for the treatment of bulimia in which a cholinesterase inhibitor is combined with at least one member selected from the group consisting of sibutramine, a pharmacologically acceptable salt thereof, an active metabolite thereof, a prodrug thereof and a solvate thereof. The pharmaceutical composition is useful in treating bulimia and depression arising from bulimia.

## Description

### Technical Field

The present invention relates to a novel combination of compounds acting on central nervous system which can be used as a pharmaceutical composition for treating bulimia and depression arising from bulimia. The present invention also relates to a method for treating bulimia and depression arising from bulimia using the above pharmaceutical composition.

### Background Art

Numbers of the patients suffering from bulimia in Japan are as many as several hundred thousands and there is also a report that females in certain ages suffer from bulimia at the rate of one out of ten. It has been also known that the patients suffer from depression as a result of bulimia.

Despite the profound symptoms of bulimia as a disease, fluoxetine is only one drug which has been approved as a treating agent for bulimia (refer, for example, to Patent Document 1) and it has been used at the dose of 60 mg per day as the optimum amount. However, the rate of improvement by that is about 60% and it is the current state that no treating method is available for the cases of bulimia where a treatment with 60 mg of fluoxetine is ineffective. Although mazindol which has been approved in Japan as an anorectic is applied for bulimia, it is a compound similar to amphetamine (a stimulant) and has side effects such as excitation, frustration, load on cardiovascular system, dysuria and others whereby an utmost carefulness is necessary for its use. With regard to sibutramine, there have been reports for its treatment for obesity (refer, for example, to Patent Document 2) and treatment for bulimia (refer, for example, to Patent Document 3).

Patent Document 1: Specification of U.S. Patent No. 5,985,322
Patent Document 2: Specification of U.S. Patent No. 5,436,272
Patent Document 3: Specification of U.S. Patent No. 6,365,633

### Disclosure of the Invention

### Problems that the Invention is to Solve

Although bulimia is a serious diseases as such, there is little choice for an effective treating method and, in addition, necessity for a drug which is also able to effectively treat depression arising from bulimia has been still present in a continuing manner even at present and the necessity is expected to further increase in future. It is an object of the present invention to provide an excellent drug which can be used for the treatment of bulimia as such.

### Means for Solving the Problems

The present inventor has conducted intensive studies for solving the above problems. During the course thereof, it has been found that patients suffered from bulimia have a lower cognitive ability to their body images, in spite of lean figure, they erroneously recognizes to be overweight for themselves. Accordingly, attention has been paid to the fact that a cholinesterase inhibitor used as a treating agent for Alzheimer's disease may recover the cognitive ability achieving an effective antagonistic action to bulimia. It was shown that, for example, donepezil hydrochloride which has been widely used as a treating agent for Alzheimer's disease improves the cognitive ability and general function of patients suffering from light to medium Alzheimer's disease by means of a double-blind clinical test (Rogers S. L., Farlow M. R., Doody R. S., Mohs R. and Friedhoff L. T. (1998) The Donepezil Study Group. A 24-week, double-blind, placebo-controlled trial for donepezil in patients with Alzheimer's disease. Neurology, 50, 135 to 145).

As a result, it has been found that the use of sibutramine and donepezil hydrochloride which is a cholinesterase inhibitor as effective ingredients of a treating agent for bulimia is very useful for patients where improvement is hardly noted in the treatment of bulimia by a drug, etc. or for patients where the treating effect is insufficient and, on the basis of the above, the present invention has been achieved. It has been also found that the use of a cholinesterase inhibitor for depression arising from bulimia is very effective whereupon the present invention has been achieved.

Thus, in one aspect, the present invention is a pharmaceutical composition for the treatment of bulimia or a pharmaceutical composition for the treatment of depression arising from bulimia in which the pharmaceutical composition is characterized in that a cholinesterase inhibitor is combined with at least one member selected from the group consisting of sibutramine, a pharmacologically acceptable salt thereof, an active metabolite thereof, a prodrug thereof and a solvate thereof. In another aspect, the present invention is a method for treating bulimia or depression arising from bulimia which is characterized in that an effective amount of the above pharmaceutical composition is administered to a patient who needs such a treatment. In a different aspect, the present invention relates to use of the above pharmaceutical composition which is characterized in that said pharmaceutical composition is used for the treatment of a patient suffering from bulimia or a patient suffering from depression arising from bulimia or for the production of a drug for the treatment of a patient suffering from bulimia or a patient suffering from depression arising from bulimia.

Accordingly, the present invention includes at least the following (1) to (17).
(1) A pharmaceutical composition for treating bulimia in which a cholinesterase inhibitor is combined with at least one member selected from the group consisting of sibutramine, a pharmacologically acceptable salt thereof, an active metabolite thereof, a prodrug thereof and a solvate thereof.
(2) A pharmaceutical composition for treating depression arising from bulimia in which a cholinesterase inhibitor is combined with at least one member selected from the group consisting of sibutramine, a pharmacologically acceptable salt thereof, an active metabolite thereof, a prodrug thereof and a solvate thereof.
(3) The composition according to (1) or (2), wherein the cholinesterase inhibitor is selected from the group consisting of donepezil, rivastigmine, galanthamine, tacrine, metrifonate, neostigmine and physostigmine as well as an enantiomer thereof, a diastereomer thereof, a tautomer thereof, a pharmacologically acceptable salt thereof, an active metabolite thereof, a prodrug thereof and a solvate thereof.
(4) The composition according to (1) or (2), wherein the cholinesterase inhibitor is donepezil, an enantiomer thereof, a pharmacologically acceptable salt thereof, an active metabolite thereof, a prodrug thereof or a solvate thereof.
(5) The composition according to (1) or (2), wherein the cholinesterase inhibitor is donepezil hydrochloride.
(6) The composition according to (1) or (2), wherein sibutramine or a pharmacologically acceptable salt thereof is sibutramine hydrochloride.
(7) The composition according to (1), wherein the pharmaceutical composition for the treatment of bulimia is a compounded agent.
(8) The composition according to (2), wherein the pharmaceutical composition for the treatment of depression arising from bulimia is a compounded agent.
(9) The composition according to (1), wherein the pharmaceutical composition for the treatment of bulimia is a kit comprising a drug containing a cholinesterase inhibitor and a drug containing at least one member selected from the group consisting of sibutramine, a pharmacologically acceptable salt thereof, an active metabolite thereof, a prodrug thereof and a solvate thereof.
(10) The composition according to (2), wherein the pharmaceutical composition for the treatment of depression arising from bulimia is a kit comprising a drug containing a cholinesterase inhibitor and a drug containing at least one member selected from the group consisting of sibutramine, a pharmacologically acceptable salt thereof, an active metabolite thereof, a prodrug thereof and a solvate thereof.
(11) A medical product comprising a drug which contains a cholinesterase inhibitor; a drug which contains at least one member selected from the group consisting of sibutramine, a pharmacologically acceptable salt thereof, an active metabolite thereof, a prodrug thereof and a solvate thereof; and a label, instructions and/or a package insert that indicate the direction for the combination of both drugs in a use for bulimia or depression arising from bulimia.
(12) A method for treating bulimia in a patient in need thereof, comprising administering an effective amount of a pharmaceutical composition containing a cholinesterase inhibitor and at least one member selected from the group consisting of sibutramine, a pharmacologically acceptable salt thereof, an active metabolite thereof, a prodrug thereof and a solvate thereof.
(13) A method for treating depression caused by bulimia in a patient in need thereof, comprising administering an effective amount of a pharmaceutical composition containing a cholinesterase inhibitor and at least one member selected from the group consisting of sibutramine, a pharmacologically acceptable salt thereof, an active metabolite thereof, a prodrug thereof and a solvate thereof.
(14) Use of a cholinesterase inhibitor and at least one substance selected from the group consisting of sibutramine, a pharmacologically acceptable salt thereof, an active metabolite thereof, a prodrug thereof and a solvate thereof for the manufacture of a drug for the treatment of bulimia.
(15) Use of a cholinesterase inhibitor and at least one substance selected from the group consisting of sibutramine, a pharmacologically acceptable salt thereof, an active metabolite thereof, a prodrug thereof and a solvate thereof for the treatment of bulimia.
(16) Use of a cholinesterase inhibitor and at least one substance selected from the group consisting of sibutramine, a pharmacologically acceptable salt thereof, an active metabolite thereof, a prodrug thereof and a solvate thereof for the manufacture of a drug for the treatment of depression arising from bulimia.
(17) Use of a cholinesterase inhibitor and at least one substance selected from the group consisting of sibutramine, a pharmacologically acceptable salt thereof, an active metabolite thereof, a prodrug thereof and a solvate thereof for the treatment of depression arising from bulimia.

### Advantages of the Invention

In accordance with the present invention, a treatment by the combination of donepezil hydrochloride with sibutramine has been effective for intractable bulimia,which is insensitive, for example, to administration of 60 mg of fluoxetine, and a significant improvement was observed. Further, after the treatment of the present invention, a wrong body image has been improved, by which the improvement of eating disorders including bulimia was exceedingly inhibited. Still further, it has been confirmed that rather dangerous anorexia and reduction in body weight by administration of sibutramine are no longer generated.
Preferred Mode for Carrying Out the Invention

Now the present invention will be illustrated in detail as follows. The embodiments mentioned hereinafter are mere examples for illustrating the present invention and are not intended to limit the invention to the embodiments. All technical terms, scientific terms and professional terms used in the present specification have the same meanings which are generally understood by persons skilled in the art to which the present invention belongs and they are used for a purpose of merely illustrating the specific embodiment and are not intended for a purpose of limitation. Any method and material which are similar or identical to those mentioned in this specification are able to be used in carrying out or in testing the present invention and, with regard to the preferred method and material thereof, the following description may be referred to. The present invention is able to be carried out in various modes so far as they are not out of the gist of the present invention.

All prior art documents, laid-open gazettes, patent gazettes and other patent documents cited in the present specification are incorporated into the present specification by reference and are able to be used for carrying out the present invention.

### [Definitions]

"Bulimia" which is a target of the present invention is a kind of eating disorder and is classified, for example, as "bulimia nervosa" according to the Diagnostic and Statistical Manual of Mental Disorders (DSM-IV) by the American Psychiatric Society. It is a disease where a compulsion where a lot of food is quickly ingested with short time is episodically repeated (excessive eating) followed by conducting a self-induced vomiting, using a purgative or a diuretic and conducting a violent exercise so as to stop an increase in body weigh (excessive eating and excretion).

"Patient" is an animal or, preferably, a mammal. "Mammal" stands for all animals classified as a mammal including human or non-human mammal (such as mouse, rat, hamster, guinea pig, rabbit, swine, dog, horse, cattle and monkey). Preferably, the mammal in the present specification is human. In that case, the term "patient" includes adult and children and also includes males and females. Children include newborn babies, small children and adolescents.

"Treatment" usually means to achieve a desired pharmacological effect and/or physiological effect. The effect is prophylactic in view of a complete or partial prevention of disease and/or symptom while it is therapeutic in view of a partial or complete cure of bad affection caused by disease and/or symptom. In this specification, "treatment" includes any treatment of a patient or, particularly, human and, for example, it includes the following (a) to (c).
(a) To prevent the onset of disease or symptom in a patient who is able to have a factor for disease or symptom but is not diagnosed to have it at present;
(b) To hinder the disease symptom or, in other words, to suppress or retard its progress;
(c) To mitigate the disease symptom or, in other words, to cause recession or disappearance of disease or symptom or to cause a reversal of symptom.

"Prodrug" means a product where "active ingredient of drug" (meaning a "drug" against a prodrug) is chemically modified to an inactive substance with an object of improvement in bioavailability or of reduction in side effects and means a drug which is metabolized to the active body in vivo to express the action. Accordingly, the term "prodrug" stands for any compound where, although an intrinsic activity is lower than the corresponding "drug", the "drug" substance is produced as a result of a spontaneous chemical reaction, enzymatic catalytic reaction or metabolic reaction when administered to a biological system. Examples of a prodrug are the compounds where amino group, hydroxyl group, carboxyl group, etc. of the drug are acylated, alkylated, phosphorylated, borated, carbonated, esterified, amidated or urethanized and are derivatives having a group which is chemically or metabolically degradable and showing the pharmaceutical activity by hydrolysis, solvolysis or degradation under a physiological condition. The exemplified groups are not inclusive but are merely typical and persons skilled in the art are able to produce various kinds of other known prodrugs by a publicly known method from cholinesterase inhibitor or sibutramine.

"Active metabolite" is a substance where an action is able to be enhanced or recognized by a drug-metabolizing enzyme.

Diagnosis of bulimia of a patient is able to be carried out using various known methods. For example, a diagnostic standard for 307.51 "bulimia nervosa" according to the DSM-IV-TR (published in 2000) which is a revised edition of the DSM-IV reads as follows.
"A. Repetition of episode of excessive eating and episode of excessive eating are characterized by the following two.
   (1) the fact of eating apparently more food, during the time zone which is clearly differentiated from others, than the amount which is ingested by most people during the same time and under the same environment
   (2) the sense that control of eating is not possible during such an episode period;
B. Inappropriate compensated act is repeated for preventing an increase in body weight such as self-induced vomiting, erroneous use of a purgative, a diuretic, an enema or other drugs, a fasting or a violent exercise.
C. Excessive eating and inappropriate compensated act happen at least two times a weak for three months in average.
D. Self-assessment is excessively affected by the influence of shape and weight of the body.
E. Disorder does not happen merely during the episodic period of anorexia nervosa.

Known cholinesterase inhibitors can be used as an effective ingredient in the present invention. Examples thereof are an acetylcholinesterase inhibitor and a butyrylcholinesterase wherein an acetylcholinesterase inhibitor is preferred.
Examples of the specific compound are as follows although they are non-limitative.
Donepezil: 1-benzyl-4-[(5,6-dimethoxy-1-indanon)-2-yl]methylpiperidine;
Rivastigmine: 3-[(S)-1-(dimethylamino)ethyl]phenyl N-ethyl-N-methylcarbamate;
Tacrine: 1,2,3,4-tetrahydro-9-acridineamine;
Galanthamine: (4aS,6R,8aS)-4a,5,9,10,11,12-hexahydro-3-methoxy-11-methyl-6H-benzofuro(3a,3,2-ef)-(2-)benzo-azepin-6-ol;
Metrifonate: dimethyl (2,2,2-trichloro-1-hydroxyethyl)phophonate;
Neostigmine: 3-(dimethylcarbamoxyphenyl) trimethylammonium; and
Physostigmine: (3aS-cis)-1,2,3,3a,8,8a-hexahydro-1,3a,8-trimethylpyrrolo[2,3-b]indol-5-ol methylcarbamate (ester).
Among them, particularly preferred are donepezil, rivastigmine and galanthamine and the most preferred is donepezil. The cholinesterase inhibitor which can be used as an effective ingredient in the present invention includes an enantiomer thereof, a diastereomer thereof, a tautomer thereof, a pharmacologically acceptable salt thereof, an active metabolite thereof, a prodrug thereof and a solvate thereof and those ones may be used as well. The cholinesterase inhibitor of the present invention includes all isomers and an isomer mixture such as geometric isomer resulted by the structure of the compound, optical isomer based on asymmetric carbon, rotational isomer, steric isomer and tautomer and is not limited to the above mentioned for convenience but any of isomers or a mixture thereof will do. Accordingly, in case of the cholinesterase inhibitor of the present invention is an optical isomer having asymmetric carbon in the molecule, or a racemate thereof, both are included in the compound without any limitation. Further, any polymorphic crystal, a single or a mixture thereof may be included in the present invention without limitation.

The cholinesterase inhibitor used as an effective ingredient in the present invention may be produced by a known method.
Donepezil can be easily produced by the methods disclosed, for example, in Japanese Patent Laid-Open No. 01/079,151, Japanese Patent No. 2,578,475, Japanese Patent No. 2,733,203, Japanese Patent No. 3,078,244 or US 4,895,841. Donepezil hydrochloride is also available as a preparation such as fine granules.
Galanthamine can be easily produced by the methods disclosed, for example, in US 4,663,318, WO 88/08708, WO 97/03987, US 6,316,439, US 6,323,195 and US 6,323,196.
Tacrine can be easily produced by the methods disclosed, for example, in US 4,631,286, US 4,695,573, US 4,754,050, WO 88/02256, US 4,835,275, US 4,839,364, US 4,999,430 and WO 97/21681.
Rivastigmine can be easily produced by the methods disclosed, for example, in EP 193,926, WO 98/26775 and WO 98/27055.
Metrifonate, neostigmine, physostigmine, etc. may be also produced by known proceses mentioned in prior art documents.

Production of sibutramine [N,N-dimethyl-1-[1-(4-chlorophenyl)cyclobutyl]-3-methylbutylamine] or a pharmacologically acceptable salt thereof (preferably, a hydrochloride) and a monohydrate thereof which is the preferred embodiment of the hydrochloride as well as use thereof for the treatment of depression are disclosed in the specification of the GB 2,098,602. There are two enantiomers in sibutramine of the present invention and the present invention is not limited to the description for the sake of convenience but includes the use of each enantiomer and a mixture of enantiomers (WO 00/054765). Therefore, in case of sibutramine of the present invention is an optical isomer having asymmetric carbon in the molecule, or a racemate thereof, both are included in the compound without any limitation. Further, any polymorphic crystal, a single or a mixture thereof may be included in the present invention without limitation. Among the monoamine reuptake inhibitors, sibutramine has a unique pharmacological profile. Due to pharmacologically active metabolites thereof (any of or both of the two methyl groups bonding to nitrogen atom), sibutramine suppresses the reuptake of all of three monoamines and, therefore, it is different from serotonin (5-HT) selective reuptake suppressors (such as fluoxetine), noradrenaline selective reuptake suppressors (such as desipramine), dopamine selective reuptake suppressors (such as bupropion) and serotonin-noradrenaline reuptake suppressors (such as venlafaxine). Due to such a unique combination of pharmacological actions, sibutramine results in an aimed effect for the treatment of anorexia nervosa, bulimia nervosa, body weight increase after smoking cessation, snacking and binge-purge syndrome.

The cholinesterase inhibitor or sibutramine of the present invention may be in a form of a pharmacologically acceptable salt thereof. For example, it is an inorganic acid such as hydrochloric acid, hydrobromic acid, hydroiodic acid, sulfuric acid and phosphoric acid and an organic acid such as p-toluenesulfonic acid, methanesulfonic acid, oxalic acid, p-bromophenylsulfonic acid, carbonic acid, succinic acid, citric acid, benzoic acid and acetic acid. Examples of the pharmacologically acceptable salt are hydrochloride, sulfate, pyrosulfate, bisulfate, sulfite, bisulfite, phosphate, monohydrogen phosphate, dihydrogen phosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, sebacate, fumarate, maleate, benzoate, hydroxybenzoate, methoxybenzoate, phthalate, terephthalate, sulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate and naphthalene-2-sulfonate although they are non-limitative. With regard to the acetylcholinesterase inhibitor of the present invention, donepezil, rivastigmine and galanthamine are particularly preferred to be in a form of hydrochloride, tartrate and hydrobromide, respectively. Sibutramine of the present invention is particularly preferred to be in a form of hydrochloride.

The cholinesterase inhibitor of the present invention, sibutramine or a pharmacologically acceptable salt thereof may be an anhydride or a solvate, if it is present. The solvatemay be either a hydrate or non-hydrate but a hydrate is preferred. To make the non-hydrate, for example, alcohol (such as methanol, ethanol or n-propanol) and dimethylformamide can be used.

Among the cholinesterase inhibitor, sibutramine and a pharmacologically acceptable salt thereof and the like of the present invention, a commercially available compound can be easily obtained from chemical manufactures, etc.

The pharmaceutical composition of the present invention is also provided as a combined administering agent of a drug containing a cholinesterase inhibitor with a drug containing at least one member selected from the group consisting of sibutramine, a pharmacologically acceptable salt thereof, an active metabolite thereof, a prodrug thereof and a solvate thereof. In the combination therapy of the present invention, each of the combined ingredients in an effective dose may be directly administered at the same time or each of them in an effective dose thereof may be administered with time intervals. Alternatively, a pharmaceutical composition is prepared by a commonly used method whereby each of the combined ingredients in an effective dose may be administered either simultaneously or with time intervals. Still alternatively, in the combination therapy of the present invention, a pharmaceutical preparation where each of the combined ingredients is just compounded is administered in an effective dose or a pharmaceutical preparation where each of the ingredients is made into a preparation to some extent followed by compounding is administered in an effective dose. A pharmaceutical product where some kinds of ingredients having same or different pharmaceutical effect are compounded in a pharmaceutical preparation is called a compounded agent or a combined preparation. It has been well known in the technical filed of the present invention that plural ingredients are combined whereby the effect is enhanced or safety is enhanced suppressing the side effect as compared with the pharmaceutical preparation comprising a single ingredient (a single preparation). It is also possible that the cholinesterase inhibitor and sibutramine are made into separate pharmaceutical preparations and they are provided as a kit. Making into pharmaceutical preparations is able to be carried out by anyone who is a person skilled in the art based on the commonly used art.

There is no particular limitation for the dosage form of the pharmaceutical composition used for the combination therapy of the present invention and it is possible to administer either orally or parenterally. In the combined use or the compounding, each of the combined or compounded ingredients may be different in view of dosage form or dose thereof. The therapeutically effective amount can be determined by degree of elapse of the diseases, age, body weight or sex of a patient and other conditions and an ability therefor is naturally included within general technical range of persons skilled in the art.

With regard to the pharmaceutical preparation, an appropriate dosage form is selected, if necessary, from the commonly known dosage forms such as tablets, coated preparation, pills, liquid, suspension, emulsion, granules, capsules, injection agent, suppository and spraying agent whereupon the preparation is able to be prepared. Among them, the preferred one is a preparation by which oral administration is possible.

With regard to a carrier used for preparing those preparations, it is possible to use commonly used excipient, binder, disintegrating agent, lubricant, coloring agent, corrigent and, if necessary, stabilizer, emulsifier, absorption accelerator, surfactant, pH adjusting agent, antiseptic, antioxidant, extender, moisturizer, surface active agent, dispersing agent, buffer, preservative, dissolving aid, anesthetizing agent, etc. and it is possible to give a pharmaceutical preparation by a common method by compounding the components which are usually used as materials for pharmaceutical preparations. Examples of nontoxic components as such are animal and plant oils such as soybean oil, beef tallow and synthetic glyceride; hydrocarbon such as liquid paraffin, squalane and solid paraffin; ester oil such as octyldodecyl myristate and isopropyl myristate; higher alcohol such as cetostearyl alcohol and behenyl alcohol; silicone resin; silicone oil; surfactant such as polyoxyethylene fatty acid ester, sorbitan fatty acid ester, glycerol fatty acid ester, polyoxyethylene sorbitan fatty acid ester, polyoxyethylene hydrogenated castor oil and a block copolymer of polyoxyethylene with polyoxypropylene; water-soluble polymer such as hydroxyethyl cellulose, polyacrylic acid, carboxyvinyl polymer, polyethylene glycol, polyvinylpyrrolidone and methyl cellulose; lower alcohol such as ethanol and isopropanol; polyhydric alcohol (polyol) such as glycerol, propylene glycol, dipropylene glycol, sorbitol and polyethylene glycol; saccharide such as glucose and sucrose; inorganic powder such as anhydrous silicic acid, aluminum magnesium silicate and aluminum silicate; inorganic salt such as sodium chloride and sodium phosphate; and pure water.

Examples of the excipient are lactose, fructose, corn starch, white sugar, glucose, mannitol, sorbitol, crystalline cellulose and silicon dioxide; examples of the binder are polyvinyl alcohol, polyvinyl ether, methyl cellulose, ethyl cellulose, acacia, tragacanth, gelatin, shellac, hydroxypropyl methyl cellulose, hydroxypropyl cellulose, polyvinylpyrrolidone, a block polymer of polypropylene glycol with polyoxyethylene and meglumine; examples of the disintegrating agent are starch, agar, gelatin powder, crystalline cellulose, calcium carbonate, sodium hydrogen carbonate, calcium citrate, dextrin, pectin and carboxymethyl cellulose calcium; examples of the lubricant are magnesium stearate, talc, polyethylene glycol, silica and hydrogenated plant oil; examples of the coloring agent are those which are allowed to add to pharmaceuticals; examples of the corrigent are cocoa powder, menthol, aromatic powder, peppermint oil, borneol and cinnamon powder. The above components may be pharmacologically acceptable salts or hydrates thereof.

In the case of oral preparations, excipient and, if necessary, binder, disintegrating agent, lubricant, coloring agent, corrigent, etc. are further added to the effective ingredients used in the preparation and then the mixture is made into diluted powder, fine particles, granules, tablets, coated tablets, capsule, etc. by a conventional method. In the case of tablets and granules, it is also possible to conduct a coating by, for example, sugar coat and others if necessary. In the case of a syrup preparation and a preparation for injection, pH adjusting agent, dissolving agent, isotonizing agent, etc. are added together, if necessary, with dissolving aid, stabilizer, etc. followed by making into the preparation by a conventional method.

As to donepezil (Aricept), its dose range is from 0.01 to 0.75 mg/kg/day.
As to tacrine (Cognex), its dose range is from 0.1 to 2.3 mg/kg/day.
As to rivastigmine (Exelon), its dose range is from 0.1 to 0.5 mg/kg/day.
As to galanthamine (Reminyl), its dosage range is from 0.05 to 1.0 mg/kg/day.
As to metrifonate (ProMem), its dosage range is from 0.1 to 2.0 mg/kg/day.
As to neostigmine, its dosage range is from 0.1 to 2.0 mg/kg/day.
As to physostigmine (Synapton), its dosage range is from 0.01 to 0.4 mg/kg/day.
As to sibutramine, its dosage range is from 0.002 to 1 mg/kg/day or, preferably, from 0.02 to 0.5 mg/kg/day.

In accordance with the present invention, there is also provided a pharmaceutical agent containing a drug which contains a cholinesterase inhibitor; a drug which contains at least one member selected from the group consisting of sibutramine, a pharmacologically acceptable salt thereof, an active metabolite thereof, a prodrug thereof and a solvate thereof; and a label, instructions and/or a package insert that indicate the direction for the combination of both drugs in a use for bulimia or depression arising from bulimia.

In that case, a drug which contains a cholinesterase inhibitor and a drug which contains at least one member selected from the group consisting of sibutramine, a pharmacologically acceptable salt thereof, an active metabolite thereof, a prodrug thereof and a solvate thereof are covered by the pharmaceutical agent according to the present invention where each of them is a separate preparation or, preferably, a separate unit dosage form.

With regard to the instructions for use of the combination of both drugs for bulimia or depression arising from bulimia, examples thereof are information concerning use method and dose such as administering amount and frequency of each drug per day, administering route, etc. When the pharmaceutical agent according to the present invention contains only one of the drugs, information concerning another drug to be used together may be mentioned in the package insert.

Examples which will be mentioned below are mere exemplifications and are only intended to illustrate the present invention in detail together with the already-mentioned preferred modes whereby they do not limit the present invention. It is also possible for persons skilled in the art to modify the present invention without deviating from the significance of the present invention and such a modification is also included within the scope of the present invention.

### Example 1

The patient of the first case (age 28; female (unmarried); company employee; height: 155 cm; body weight: 52 kg) has started in dieting from about 18 year age. Here body weight which was 55.0 kg at that time decreased to 46.5 kg after two months. However, after maintenance for one month, excessive eating and vomiting began and there was a state where vomiting and excessive eating for not less than once daily were unable to be suppressed. After that, she graduated from her college when she was 23 years age and employed by the company but excessive eating and vomiting still continued.

She already received a drug therapy with Paxil, fluvoxamine, diazepam, tricyclic antidepressant, sodium valproate, Rivotril, Risperdal, etc. and a spiritual therapy at three neurological hospitals but no improvement was noted. At the first medical examination, a depressive state was noted and excessive eating and vomiting were repeated once daily on weekdays and two to three time daily on holidays. There was no particular problem in biochemical data for her blood.

When 10 mg of sibutramine and 5 mg of Aricept were administered to her, frequency of excessive eating reduced from three days thereafter, i.e., one excessive eating and vomiting every three days until one week thereafter and, on the 14th day and after that, no excessive eating was noted together with improvement in depression. After one month, although excessive eating and vomiting were noted once every twenty days, an excessive eating amount became about one-half and that was the same after three months as well. As to the side effect, insomnia and shortening of sleeping time were noted but no more side action was noted. There was no problem in the biochemical data of here blood after three months and ECG was within a normal range as well.

### Example 2

The patient of the second case (21 years age; female university student (unmarried); height: 161 cm; body weight: 58 kg) had a poor body image and showed excessive eating and vomiting for two to three times a day and suppression.

An excessive eating started when she was the third year class (age: 17) of high school and it is still continuing. At the first medical examination, she was unable to go to university. Firstly, 20 mg of fluoxetine was administered for two weeks but no improvement in excessive eating was noted. Then, fluoxetine was increased to 40 mg but no improvement was noted yet. After four weeks from the initiation of the therapy, fluoxetine was increased up to 60 mg and administered for two weeks but no decrease in amount and frequency of excessive eating were noted. No change in depression was noted as well (at the time of six weeks from the initiation of the therapy).

Then administration of the drug was stopped for two weeks and, at the time of eight weeks from the initiation of the therapy, administration of 10 mg of Aricept and 10 mg of sibutramine was started whereupon frequency of excessive eating reduced after one week (after nine weeks from the initiation of the therapy) and, after two weeks (after ten weeks from the initiation of the therapy), excessive eating stopped. Suppression was improved as well. Even after one half year, no recurrence was noted, body weight was maintained, body image was also improved and she became to be able to judge for the fact of "being fat and being slim" in the same manner as healthy adults do.

## Claims

1. A pharmaceutical composition for treating bulimia in which a cholinesterase inhibitor is combined with at least one member selected from the group consisting of sibutramine, a pharmacologically acceptable salt thereof, an active metabolite thereof, a prodrug thereof and a solvate thereof.

2. A pharmaceutical composition for treating depression arising from bulimia in which a cholinesterase inhibitor is combined with at least one member selected from the group consisting of sibutramine, a pharmacologically acceptable salt thereof, an active metabolite thereof, a prodrug thereof and a solvate thereof.

3. The composition according to claim 1 or 2, wherein the cholinesterase inhibitor is selected from the group consisting of donepezil, rivastigmine, galanthamine, tacrine, metrifonate, neostigmine and physostigmine as well as an enantiomer thereof, a diastereomer thereof, a tautomer thereof, a pharmacologically acceptable salt thereof, an active metabolite thereof, a prodrug thereof and a solvate thereof.

4. The composition according to claim 1 or 2, wherein the cholinesterase inhibitor is donepezil, an enantiomer thereof, a pharmacologically acceptable salt thereof, an active metabolite thereof, a prodrug thereof or a solvate thereof.

5. The composition according to claim 1 or 2, wherein the cholinesterase inhibitor is donepezil hydrochloride.

6. The composition according to claim 1 or 2, wherein sibutramine or a pharmacologically acceptable salt thereof is sibutramine hydrochloride.

7. The composition according to claim 1, wherein the pharmaceutical composition for treating bulimia is a compounded agent.

8. The composition according to claim 2, wherein the pharmaceutical composition for the treatment of depression arising from bulimia is a compounded agent.

9. The composition according to claim 1, wherein the pharmaceutical composition for treating bulimia is a kit comprising a drug containing a cholinesterase inhibitor and a drug containing at least one member selected from the group consisting of sibutramine, a pharmacologically acceptable salt thereof, an active metabolite thereof, a prodrug thereof and a solvate thereof.

10. The composition according to claim 2, wherein the pharmaceutical composition for treating depression arising from bulimia is a kit comprising a drug containing a cholinesterase inhibitor and a drug containing at least one member selected from the group consisting of sibutramine, a pharmacologically acceptable salt thereof, an active metabolite thereof, a prodrug thereof and a solvate thereof.

11. A medical product comprising a drug which comprises a cholinesterase inhibitor; a drug which comprises at least one member selected from the group consisting of sibutramine, a pharmacologically acceptable salt thereof, an active metabolite thereof, a prodrug thereof and a solvate thereof; and a label, instructions and/or a package insert that indicate the direction for the combination of both drugs in a use for bulimia or depression arising from bulimia.

12. A method for treating bulimia in a patient in need thereof, comprising administering an effective amount of a pharmaceutical composition containing a cholinesterase inhibitor and at least one member selected from the group consisting of sibutramine, a pharmacologically acceptable salt thereof, an active metabolite thereof, a prodrug thereof and a solvate thereof.

13. A method for treating depression caused by bulimia in a patient in need thereof, comprising administering an effective amount of a pharmaceutical composition containing a cholinesterase inhibitor and at least one member selected from the group consisting of sibutramine, a pharmacologically acceptable salt thereof, an active metabolite thereof, a prodrug thereof and a solvate thereof.

14. Use of a cholinesterase inhibitor and at least one substance selected from the group consisting of sibutramine, a pharmacologically acceptable salt thereof, an active metabolite thereof, a prodrug thereof and a solvate thereof for the manufacture of a drug for the treatment of bulimia.

15. Use of a cholinesterase inhibitor and at least one substance selected from the group consisting of sibutramine, a pharmacologically acceptable salt thereof, an active metabolite thereof, a prodrug thereof and a solvate thereof for the treatment of bulimia.

16. Use of a cholinesterase inhibitor and at least one substance selected from the group consisting of sibutramine, a pharmacologically acceptable salt thereof, an active metabolite thereof, a prodrug thereof and a solvate thereof for the manufacture of a drug for the treatment of depression arising from bulimia.

17. Use of a cholinesterase inhibitor and at least one substance selected from the group consisting of sibutramine, a pharmacologically acceptable salt thereof, an active metabolite thereof, a prodrug thereof and a solvate thereof for the treatment of depression arising from bulimia.
